# EUROPÄISCHE PATENTSCHRIFT

(11) **EP 0 692 245 B1**
(45) Veröffentlichungstag und Bekanntmachung des Hinweises auf die Patenterteilung: **02.05.1997**
(21) Anmeldenummer: 95101868.8
(22) Anmeldetag: 11.02.1995
(51) Int. Cl.: A61K 7/13

(54) **Mittel zum oxidativen Färben von Haaren auf der Basis von 4,5-Diaminopyrazolen und m-Phenylendiaminderivaten**
Oxidation hair dye containing 4,5-diaminopyrazole derivatives and m-phenylendiamine derivatives
Colorant d'oxydation pour les cheveux à base de diaminopyrazole-4,5 et dérivés de m-phenylenediamine

(30) Priorität: 28.06.1994 DE 4422603
(43) Veröffentlichungstag der Anmeldung: 17.01.1996
(73) Patentinhaber: Wella Aktiengesellschaft, 64274 Darmstadt (DE)
(72) Erfinder: Löwe, Isolde, D-64625 Bensheim (DE); Weinges, Alexa, Dr., D-69121 Heidelberg (DE); Balzer, Wolfgang R., Dr., D-64665 Alsbach (DE); Gerstung, Stefan, Dr., D-64354 Reinheim (DE)

(56) Entgegenhaltungen:
- EP-A- 0 375 977
- WO-A-94/08970
- DD-A- 208 298

## Beschreibung

Gegenstand der Erfindung sind Mittel zur oxidativen Färbung von Haaren auf der Basis einer Entwicklersubstanz-Kupplersubstanz-Kombination, welche bestimmte 4,5-Diaminopyrazolderivate als Entwicklersubstanzen sowie bestimmte m-Phenylendiaminderivate als Kupplersubstanzen enthalten.

Auf dem Gebiet der Haarfärbung haben Oxidationsfarbstoffe eine wesentliche Bedeutung erlangt. Die Färbung entsteht hierbei durch Reaktion bestimmter Entwicklersubstanzen mit bestimmten Kupplersubstanzen in Gegenwart eines geeigneten Oxidationsmittels.

Als Entwicklersubstanzen werden insbesondere 2,5-Diaminotoluol, 2,5-Diaminophenylethylalkohol, p-Aminophenol und 1,4-Diaminobenzol eingesetzt. Von den vorzugsweise verwendeten Kupplersubstanzen sind Resorcin, 4-Chlorresorcin, 1-Naphthol, 3-Aminophenol, 5-Amino-2-methylphenol und Derivate des m-Phenylendiamins zu nennen.

An Oxidationsfarbstoffe, die zur Färbung menschlicher Haare verwendet werden, werden zahlreiche besondere Anforderungen gestellt. So müssen sie in toxikologischer und dermatologischer Hinsicht unbedenklich sein und Färbungen in der gewünschten Intensität ermöglichen. Ferner wird für die erzielten Haarfärbungen eine gute Licht-, Dauerwell-, Säure- und Reibechtheit gefordert. Auf jeden Fall aber müssen solche Haarfärbungen ohne Einwirkung von Licht, Reibung und chemischen Mitteln über einen Zeitraum von mindestens 4 bis 6 Wochen stabil bleiben. Außerdem ist es erforderlich, daß durch Kombinationen geeigneter Entwickler- und Kupplerkomponenten eine breite Palette verschiedener Farbnuancen erzeugt werden kann.

Zur Erzielung modischer Nuancen im Blaubereich werden vor allem p-Phenylendiaminderivate, allein oder im Gemisch mit anderen Entwicklersubstanzen, in Kombination mit geeigneten Kupplersubstanzen eingesetzt.

Gegen den für den Blaubereich der Farbskala bisher hauptsächlich eingesetzten Entwickler p-Phenylendiamin wurden in letzter Zeit Bedenken in Bezug auf die physiologische Verträglichkeit erhoben, während die in neuerer Zeit empfohlenen Entwicklersubstanzen, wie zum Beispiel Pyrimidinderivate, in färberischer Hinsicht nicht völlig zufriedenstellen können. Die in der DE-PS 2 160 317 beschriebenen Pyrazolderivate, wie zum Beispiel das 3-Amino-1-phenyl-2-pyrazolon-5, färben ebenfalls Haare nur in sehr geringen, für die Haarfärbepraxis unbrauchbaren, Farbtiefen an.

Die aus der DE-OS 38 43 892 bekannten Diaminopyrazole ergeben in Kombination mit üblichen Kupplern hautpsächlich intensive Rottöne, so daß zur Erzielung von natürlichen Färbungen Kombinationen von Diaminopyrazolen mit anderen Entwicklern vom p-Phenylendiamintyp eingesetzt werden müssen.

Es bestand daher die Aufgabe, ein Oxidationshaarfärbemittel auf der Basis einer Entwicklersubstanz-Kupplersubstanz-Kombination zur Verfügung zu stellen, das physiologisch sehr gut verträglich ist, brilliante blaue Farbtöne mit einer hohen Farbtiefe ergibt und insbesondere auch dazu geeignet ist, natürliche Färbungen zur Verfügung zu stellen.

Hierzu wurde nun gefunden, daß durch ein Mittel zur oxidativen Färbung von Haaren auf der Basis einer Entwicklersubstanz-Kupplersubstanz-Kombination das als Entwicklersubstanz mindestens ein Diaminopyrazol der allgemeinen Formel (I) 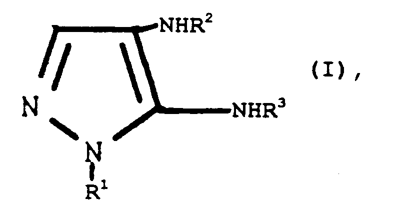 in der R¹ Wasserstoff, eine Alkylgruppe mit 1 bis 6 Kohlenstoffatomen, eine Hydroxyalkylgruppe mit 2 bis 4 Kohlenstoffatomen oder einen substituierten oder unsubstituierten Benzylrest darstellt und R² und R³ gleich oder verschieden sind und Wasserstoff, eine Alkylgruppe mit 1 bis 6 Kohlenstoffatomen oder eine Hydroxyalkylgruppe mit 2 bis 4 Kohlenstoffatomen bedeuten; und als Kupplersubstanz mindestens ein m-Phenylendiamin der allgemeinen Formel (II) 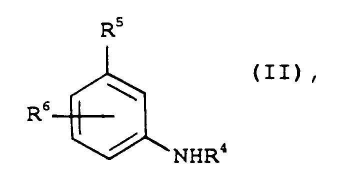 in der R⁴ gleich -CONH₂ oder -SO₂CH₃ ist, R⁵ gleich NR⁷R⁸ ist, wobei R⁷ und R⁸ gleich oder verschieden sein können und Wasserstoff oder eine Alkylgruppe mit 1 bis 4 Kohlenstoffatomen darstellen, und R⁶ Wasserstoff, eine Alkoxygruppe mit 1 bis 4 Kohlenstoffatomen oder eine Alkylgruppe mit 1 bis 4 Kohlenstoffatomen bedeutet; oder deren physiologisch verträgliche, wasserlösliche Salze enthält, die gestellte Aufgabe in hervorragender Weise gelöst wird.

In dem Haarfärbemittel sollen die Entwicklersubstanzen der Formel (I), von denen die 4,5-Diamino-1-alkylpyrazole, das 4,5-Diamino-1-benzylpyrazol, das 4,5-Diamino-1-(4'-methyl-benzyl)-pyrazol, sowie das 4,5-Diamino-1-(4'-chlorbenzyl)pyrazol bevorzugt sind, und die Kupplersubstanzen der Formel (II), von denen N-(3-Dimethylamino)phenylharnstoff und 3-(N-Methylsulfonyl)amino-N,N-dimethylanilin bevorzugt sind, jeweils in einer Menge von etwa 0,01 bis 4,0 Gewichtsprozent, vorzugsweise in einer Menge von 0,1 bis 3,0 Gewichtsprozent, enthalten sein.

Obwohl die vorteilhaften Eigenschaften der hier beschriebenen Entwicklersubstanzen es nahelegen, diese als alleinige Entwicklersubstanz zu verwenden, ist es selbstverständlich auch möglich, die Entwicklersubstanzen der Formel (I) gemeinsam mit anderen bekannten Entwicklersubstanzen, wie zum Beispiel 1,4-Diaminobenzol, 2,5-Diaminotoluol oder 2,5-Diaminophenylethanol einzusetzen.

Ebenfalls ist es möglich, neben den Kupplersubstanzen der Formel (II) weitere übliche Kupplersubstanzen, wie zum Beispiel Resorcin, 4-Chlorresorcin, 4,6-Dichlorresorcin, 2-Methylresorcin, 2-Amino-4-(2'-hydroxyethyl)amino-anisol, m-Phenylendiamin, 5-Amino-2-methylphenol, 2,4-Diaminophenoxy-ethanol, 4-Amino-2-hydroxyphenoxyethanol, 1-Naphthol,3-Aminophenol, 3-Amino-2-methylphenol, 4-Hydroxy-1,2-methylendioxybenzol, 4-Amino-1,2-methylendioxybenzol, 2,4-Diamino-5-methylphenetol, 4-Hydroxy-indol und 3,5-Diamino-2,6-dimethoxypyridin in dem erfindungsgemäßen Haarfärbemittel einzusetzen.

Die Kupplersubstanzen und Entwicklersubstanzen können in dem Haarfärbemittel jeweils einzeln oder im Gemisch miteinander enthalten sein.

Die Gesamtmenge der in dem hier beschriebenen Haarfärbemittel enthaltenen Entwicklersubstanz-Kupplersubstanz-Kombination beträgt 0,02 bis 8,0 Gewichtsprozent, wobei eine Menge von 0,2 bis 6,0 Gewichtsprozent bevorzugt ist.

Die Entwicklerkomponente wird im allgemeinen in etwa äquimolaren Mengen, bezogen auf die Kupplerkomponente, eingesetzt. Es ist jedoch nicht nachteilig, wenn die Entwicklerkomponente in einem gewissen Überschuß oder Unterschuß gegenüber der Kupplerkomponente vorhanden ist.

Weiterhin kann das erfindungsgemäße Haarfärbemittel zusätzlich andere Farbkomponenten, beispielsweise 6-Amino-2-methyl-phenol oder 2-Amino-5-methyl-phenol, sowie ferner übliche direktziehende Farbstoffe, zum Beispiel Triphenylmethanfarbstoffe wie C. I. Basic Violet 14 (C. I. 42 510) und C. I. Basic Violet 2 (C. I. 42 520), aromatische Nitrofarbstoffe wie 2-Amino-4,6-dinitrophenol, 2-Amino-5-(2'-hydroxyethyl)aminonitrobenzol und 2-Methylamino-5-bis-(2'-hydroxyethyl)amino-nitrobenzol, Azofarbstoffe wie C. I. Acid Brown 4 (C. I. 14 805) und Dispersionsfarbstoffe wie beispielsweise 1,4-Diaminoanthrachinon und 1,4,5,8-Tetraaminoanthrachinon enthalten. Die Haarfärbemittel können diese Farbkomponenten in einer Menge von etwa 0,1 bis 4,0 Gewichtsprozent enthalten.

Selbstverständlich können die Kuppler- und Entwicklersubstanzen sowie die anderen Farbkomponenten -sofern es Basen sind- auch in Form der physiologisch verträglichen Salze mit organischen oder anorganischen Säuren, wie beispielsweise Salzsäure oder Schwefelsäure, beziehungsweise -sofern sie aromatische OH-Gruppen besitzenin Form der Salze mit Basen, zum Beispiel als Alkaliphenolate, eingesetzt werden.

Darüber hinaus können in dem Haarfärbemittel noch weitere übliche kosmetische Zusätze, beispielsweise Antioxidantien wie Ascorbinsäure, Thioglykolsäure oder Natriumsulfit, sowie Parfümöle, Komplexbildner, Netzmittel, Emulgatoren, Verdicker und Pflegestoffe enthalten sein.

Die Zubereitungsform des neuen Haarfärbemittels kann beispielsweise eine Lösung, insbesondere eine wäßrige oder wäßrig-alkoholische Lösung sein. Die besonders bevorzugten Zubereitungsformen sind jedoch eine Creme, ein Gel oder eine Emulsion.

Ihre Zusammensetzung stellt eine Mischung der Farbstoffkomponenten mit den für solche Zubereitungen üblichen Zusätzen dar.

Übliche Zusätze in Lösungen, Cremes, Emulsionen oder Gelen sind zum Beispiel Lösungsmittel wie Wasser, niedere aliphatische Alkohole, beispielsweise Ethanol, Propanol, Isopropanol und Glycerin, oder Glykole, beispielsweise 1,2-Propylenglykol, weiterhin Netzmittel oder Emulgatoren aus den Klassen der anionischen, kationischen, amphoteren oder nichtionogenen oberflächenaktiven Substanzen, beispielsweise Fettalkoholsulfate, oxethylierte Fettalkoholsulfate, Alkylsulfonate, Alkylbenzolsulfonate, Alkyltrimethylammoniumsalze, Alkylbetaine, oxethylierte Fettalkohole, oxethylierte Nonylphenole, Fettsäurealkanolamide, oxethylierte Fettsäureester, ferner Verdicker wie höhere Fettalkohole, Stärke, Cellulosederivate, Vaseline, Paraffinöl und Fettsäuren sowie außerdem Pflegestoffe wie kationische Harze, Lanolinderivate, Cholesterin, Pantothensäure und Betain. Die erwähnten Bestandteile werden in den für solche Zwecke üblichen Mengen verwendet, zum Beispiel die Netzmittel und Emulgatoren in Konzentrationen von etwa 0,5 bis 30 Gewichtsprozent, die Verdicker in einer Menge von etwa 0,1 bis 25 Gewichtsprozent und die Pflegestoffe in einer Konzentration von etwa 0,1 bis 5,0 Gewichtsprozent.

Je nach Zusammensetzung kann das erfindungsgemäße Haarfärbemittel schwach sauer, neutral oder alkalisch reagieren. Insbesondere weist es einen pH-Wert von 6,5 bis 11,5 auf, wobei die Einstellung vorzugsweise mit Ammoniak erfolgt. Es können aber auch organische Amine, zum Beispiel Monoethanolamin und Triethanolamin, oder auch anorganische Basen wie Natriumhydroxid und Kaliumhydroxid Verwendung finden.

Für die Anwendung zur oxidativen Färbung von Haaren vermischt man das vorstehend beschriebene Haarfärbemittel üblicherweise unmittelbar vor dem Gebrauch mit einem Oxidationsmittel und trägt eine für die Haarfärbebehandlung ausreichende Menge, je nach Haarfülle im allgemeinen etwa 60 bis 200 Gramm, dieses Gemisches auf das Haar auf.

Als Oxidationsmittel zur Entwicklung der Haarfärbung kommen hauptsächlich Wasserstoffperoxid oder dessen Additionsverbindungen an Harnstoff, Melamin oder Natriumborat in Form einer 3- bis 12-prozentigen, vorzugsweise einer 3- bis 6-prozentigen wäßrigen Lösung, aber auch Luftsauerstoff in Betracht. Wird eine 4- bis 6-prozentige Wasserstoffperoxidlösung als Oxidationsmittel verwendet, so beträgt das Gewichtsverhältnis zwischen Haarfärbemittel und Oxidationsmittel vorzugsweise 5 : 1 bis 1 : 2, wobei ein Verhältnis von 1 : 1 besonders bevorzugt ist. Größere Mengen an Oxidationsmittel werden vor allem bei höheren Farbstoffkonzentrationen im Haarfärbemittel oder wenn gleichzeitig eine stärkere Bleichung der Haare beabsichtigt ist, verwendet. Man läßt das Gemisch bei 15 bis 50 Grad Celsius etwa 10 bis 45 Minuten lang, vorzugsweise 30 Minuten lang, auf das Haar einwirken, spült sodann das Haar mit Wasser aus und trocknet es. Gegebenenfalls wird im Anschluß an diese Spülung mit einem Shampoo gewaschen und eventuell mit einer schwachen organischen Säure, wie zum Beispiel Zitronensäure oder Weinsäure, nachgespült. Anschließend wird das Haar getrocknet.

Die Entwicklersubstanzen der Formel (I) und die Kupplersubstanzen der Formel (II) sollen in dem Haarfärbemittel entweder als freie Basen oder in Form ihrer physiologisch verträglichen Salze mit anorganischen oder organischen Säuren wie zum Beispiel Salzsäure, Schwefelsäure, Phosphorsäure, Essigsäure, Propionsäure, Milchsäure oder Zitronensäure, eingesetzt werden. Die Verbindungen der Formel (I) und (II) sind gut in Wasser löslich, sie weisen außerdem eine ausgezeichnete Lagerstabilität, insbesondere als Bestandteil der hier beschriebenen Haarfärbemittel auf.

Das erfindungsgemäße Haarfärbemittel erlaubt die Erzeugung modischer Blautöne mit außerordentlicher Farbtiefe und hoher Stabilität. Die sehr guten färberischen Eigenschaften der Haarfärbemittel gemäß der vorliegenden Anmeldung zeigen sich weiterhin darin, daß diese Mittel eine problemlose Anfärbung von ergrauten, chemisch nicht vorgeschädigten Haaren mit guter Deckkraft ermöglichen.

Die Herstellung der erfindungsgemäßen Kupplersubstanzen kann in Analogie zu den in der DE-OS 24 49 101 und der DE-PS 1 810 191 beschriebenen Herstellungsverfahren erfolgen. Danach können die Harnstoffderivate der allgemeinen Formel (II) durch Umsetzung von Anilinderivaten mit Kaliumisocyanat dargestellt werden, während die Methylsulfonamide der Formel (II) durch Umsetzung der entsprechenden 3-Nitroaniline mit Methansulfosäurechlorid und anschließende Reduktion erhältlich sind.

Verfahren zur Herstellung der in dem erfindungsgemäßen Haarfärbemittel verwendeten Entwicklersubstanzen der Formel (I) sind aus der Literatur, beispielsweise der DE-OS 3 843 892 sowie H. Dorn et al., Liebigs Annalen der Chemie 707 (1967), Seite 141 bis 146 und H. Dorn et al., Liebigs Annalen der Chemie 717 (1968), Seite 118 bis 123, bekannt.

Die Darstellung von am Benzylring substituierten 4,5-Diamino-1-benzylpyrazolen (VI) ist nach dem in der DE-OS 42 34 887 beschriebenen Verfahren durch Umsetzung von 3,5-Dibrom-4-nitropyrazol (III) mit den entsprechenden substituierten Benzylhalogeniden nach folgendem Schema ebenfalls in einfacher Weise möglich. 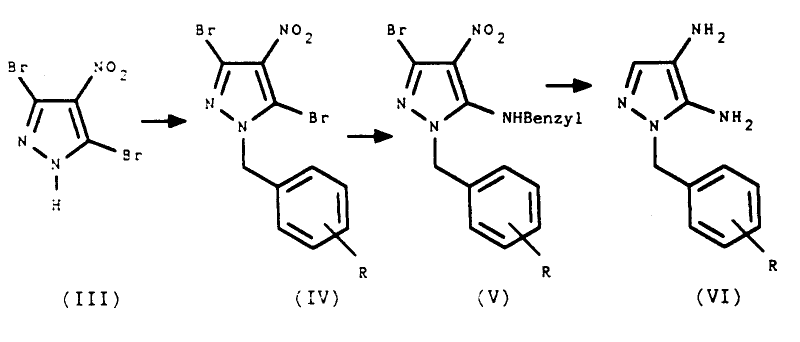 [vorzugsweise mit R = H; Halogen; C₁-C₆-Alkyl, C₁-C₆-Hydroxyalkyl; C₂-C₆-Dihydroxyalkyl; C₁-C₆-Alkoxy; Hydroxy; Cyano; Monohydroxy-(C₁-C₄)alkylamino; Dihydroxy-(C₁-C₄)alkylamino; Amino; C₁-C₆-Monoalkylamino; (C₁-C₆)-Dialkylamino].

Die nachfolgenden Beispiele sollen den Gegenstand der Erfindung näher erläutern ohne ihn darauf zu beschränken.

### Herstellungsbeispiele

### Beispiel 1: Synthese von 1-Benzyl-3,5-dibrom-4-nitropyrazolen (IV)

### Allgemeine Vorschrift

Zu 1,75 g (70 mmol) Natriumhydrid (96%) in 150 ml absolutem Dimethylformamid (DMF) werden über einen Zeitraum von 1 Stunde 19,0 g (70 mmol) 3,5-Dibrom-4-nitropyrazol in 90 ml absolutem DMF getropft. Man beobachtet eine Erwärmung der Reaktionslösung und starke Gasentwicklung. Sobald sich die Gasentwicklung gelegt hat, liegt eine klare orangefarbene Lösung vor. Nun werden 70 mmol Benzylhalogenid in 30 ml DMF zugetropft und es wird 3 Stunden auf 80 Grad Celsius erhitzt. Das Lösungsmittel wird im Vakuum abdestilliert und der Rückstand aus Methylenchlorid umkristallisiert.

### I. 3,5-Dibrom-1-(4'methylbenzyl)-4-nitro-pyrazol

9,8 g (70 mmol) 4-Methyl-benzylchlorid werden nach der vorstehend beschriebenen allgemeinen Vorschrift umgesetzt.

### Ausbeute:

18 g (68 Prozent der Theorie) 3,5-Dibrom-1-(4'-methylbenzyl)-4-nitro-pyrazol mit einem Schmelzpunkt von 108 Grad Celsius.
¹H-NMR(300MHz,DMSO-d₆):
- δ =: 2,28 ppm (s; 3H; -CH₃);
5,45 ppm (s; 2H; -CH₂-);
7,18 ppm (m; 4H; C₆H₅-H)
- MS (70 eV):: m/e = 375 (M^{+·})

### II. 3,5-Dibrom-1-(4'-chlorbenzyl)-4-nitro-pyrazol

11,3 g (70 mmol) 4-Chlor-benzylchlorid werden nach der vorstehend beschriebenen allgemeinen Vorschrift zur Reaktion gebracht.

### Ausbeute:

25 g (91 Prozent der Theorie) 3,5-Dibrom-1-(4'-chlorbenzyl)-4-nitro-pyrazol mit einem Schmelzpunkt von 109 Grad Celsius.
¹H-NMR(300 MHz;DMSO-d₆):
- δ =: 5,51 ppm (s; 2H; -CH₂);
7,28 ppm (d; J = 8,4 Hz; 2H; C₆H₅-3H und C₆H₅-5H);
7,75 ppm (d; J = 8,4 Hz; 2H; C₆H₅-2H und C₆H₅-6H)
- MS (70 eV):: m/e = 395 (M^{+·})

### Beispiel 2: Synthese von 5-Benzylamino-1-benzyl-3-brom-4-nitro-pyrazolen (V)

### I. 5-Benzylamino-3-brom-1-(4'-methylbenzyl)-4-nitropyrazol

15 g (40 mmol) 3,5-Dibrom-1-(4'-methylbenzyl)-4-nitro-pyrazol werden in 120 ml Ethanol mit 15 ml Benzylamin versetzt. Nach 1 Stunde Erwärmen wird auf Eis gegossen, abgesaugt und aus Ethanol umkristallisiert.

### Ausbeute:

10,0 g (62 Prozent der Theorie) 5-Benzylamino-3-brom-1-(4'-methylbenzyl)-4-nitropyrazol mit einem Schmelzpunkt von 108 Grad Celsius.
¹H-NMR(300MHz,DMSO-d₆):
- δ =: 2,29 ppm (s; 3H; -CH₃);
4,54 ppm (d;J = 6,4 Hz; 2H; NH-CH₂-; nach D₂O-Austausch s);
5,51 ppm (s; 2H; 1-CH₂).
7,15-7,35 ppm(m; 9H; C₆H₅-H);
8,00 ppm (t;J=6,4 Hz; 1H; NH; tauscht mit D₂O aus)
- MS (70 eV):: m/e = 400 (M^{+·})

### II. 5-Benzylamino-3-brom-1-(4'-chlorbenzyl)-4-nitropyrazol

9,88 g (25 mmol) 3,5-Dibrom-1-(4'-chlorbenzyl)-4-nitropyrazol werden in 50 ml Ethanol mit 15 ml Benzylamin 1 Stunde erwärmt. Man gießt auf Eis, saugt ab und kristallisiert aus Ethanol um.

### Ausbeute:

8 g (76 Prozent der Theorie) 5-Benzylamino-3-brom-1-(4'-chlorbenzyl)-4-nitro-pyrazol mit einem Schmelzpunkt von 139 bis 141 Grad Celsius.
¹H-NMR(300MHz,DMSO-d₆):
- δ =: 4,54 ppm (d;J = 5,4 Hz; 2H; NH-CH₂; s nach D₂O-Austausch);
5,23 ppm (s; 2H; 1-CH₂-);
7,10-7,38 ppm (m; 9H; C₆H₅-H);
8,00 (t; 1H; NH; tauscht mit D₂O aus).

### Beispiel 3: Synthese von 1-Benzyl-4,5-diaminopyrazolen der allgemeinen Formel (VI)

### Allgemeine Vorschrift: Hydrierung von 5-Benzylamino-1-benzyl-3-brom-4-nitropyrazolen (V)

Die angegebene Menge des 5-Benzylamino-1-benzyl-3-brom-4-nitropyrazols (V) wird mit 130 ml Ethanol und 2 Spatelspitzen eines Palladium/Aktivkohle-Katalysators (10%) in den Autoklaven (250 ml) überführt und während des angegebenen Zeitraumes bei 50 bar Wasserstoffatmosphäre und Raumtemperatur gerührt. Nach Reaktionsende saugt man mit einer Wasserstrahlpumpe die Reaktionsmischung in einen Glaskolben und filtriert sofort über eine Glasfritte. Das Filtrat wird mit einer äquimolaren Mengen Schwefelsäure (97%) versetzt.

### I. 4,5-Diamino-1-(4'-methylbenzyl)pyrazol

3,0 g (7,5 mmol) 5-Benzylamino-3-brom-1-(4'-methylbenzyl)-4-nitropyrazol werden 4 Stunden analog der vorstehenden allgemeinen Vorschrift hydriert. Man erhält 1,90 g (84 Prozent der Theorie) 4,5-Diamino-1-(4'-methylbenzyl)pyrazol-hydrosulfat mit einem Schmelzpunkt von 163 bis 167 Grad Celsius (unter Zersetzung).
¹H-NMR(300 MHz, DMSO-d₆):
- δ =: 2,26 ppm (s; 3H; -CH₃);
5,12 ppm (s; 2H; -CH₂-);
5,8-6,7 ppm (s; br; 6H; NH₂; H₂SO₄; tauscht mit D₂O aus);
7,05 bis 7,21 ppm (m; 4H; C₆H₅-H);
7,31 ppm (s; 1H, 3-H)
- MS (70 eV):: m/e = 202 (M^{+·})

### II. 4,5-Diamino-1-(4'-chlorbenzyl)-pyrazol

3,15 (7,5 mmol) 5-Benzylamino-3-brom-1-(4'-chlorbenzyl)-4-nitropyrazol werden 3 Stunden nach Vorschrift hydriert. Man erhält 2 g (82 Prozent der Theorie) 4,5-Diamino-1-(4'-chlorbenzyl)-pyrazol-hydrosulfat mit einem Schmelzpunkt von 188 Grad Celsius (unter Zersetzung).
¹H-NMR(300 MHz,DMSO-d₆):
- δ =: 5,12 ppm (s; 2H; -CH₂);
6,0-6,8 ppm (s; br; 6H; NH₂; H₂SO₄; tauscht mit D₂O aus)
7,13-7,39 ppm (m; 5H; 3-H und C₆H₅-H)
- MS 870 (eV):: m/e = 222 (M^{+·})

Für alle ¹H-NMR-Spektren gilt:
s = Singulett; d = Dublett; t = Triplett; m = Multiplett; br = breites Signal

### Beispiele für Haarfärbemittel

### Beispiel 4-8: Haarfärbemittel

Es werden Haarfärbelösungen folgender Zusammensetzung hergestellt:
- 0,025 mol: Entwickler nach Tabelle 1
- 0,025 mol: Kuppler nach Tabelle 1
- 10,00 g: Laurylakohol-diglycolethersulfat-Natriumsalz (28-prozentige wäßrige Lösung
- 10,00 g: Ammoniak (22-prozenprozentige wäßrige Lösung)
- ad 100,000 g: Wasser
Man vermischt kurz vor dem Gebrauch 50 g des vorstehend beschriebenen Haarfärbemittels mit 50 g Wasserstoffperoxidlösung (6-prozentig) und läßt die Mischung 30 Minuten bei 40 Grad Celsius auf blonde Naturhaare einwirken. Das Haar wird mit Wasser gespült und getrocknet. Die resultierenden Färbungen sind in Tabelle 1 dargestellt.

Alle Angaben stellen, soweit nichts anderes angegeben ist, Gewichtsprozente dar.

**Tabelle 1**

| Beispiel | Entwickler | Kuppler | Farbe |
|---|---|---|---|
| 4 | 4,5-Diamino-1-isopropylpyrazol | N-[(3-Dimethylamino)phenyl]-harnstoff | blau |
| 5 | 4,5-Diamino-1-isopropylpyrazol | N-(3-Amino-4-methoxyphenyl)-harnstoff | dunkel-violett |
| 6 | 4,5-Diamino-1-(4'-methylbenzyl)pyrazol | 3-(N-Methylsulfonylamino)-N,N-dimethylanilin | blau |
| 7 | 4,5-Diamino-1-(4'-methylbenzyl)pyrazol | N-(3-Amino-4-methylphenyl)-harnstoff | violett |
| 8 | 4,5-Diamino-1-(4'-chlorbenzyl)pyrazol | 2-Amino-4-(N-methylsulfonyl)amino-anisol | hell-violett |

## Patentansprüche

1. Mittel zur oxidativen Färbung von Haaren auf der Basis einer Entwicklersubstanz-Kupplersubstanz-Kombination, dadurch gekennzeichnet, daß es als Entwicklersubstanz mindestens ein Diaminopyrazol der allgemeinen Formel (I) 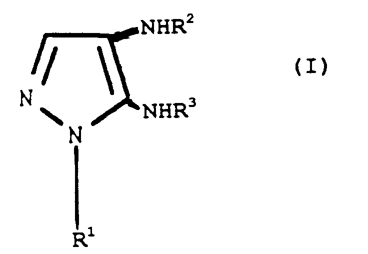 in der R¹ Wasserstoff, eine Alkylgruppe mit 1 bis 6 Kohlenstoffatomen, eine Hydroxyalkylgruppe mit 2 bis 4 Kohlenstoffatomen oder einen substituierten oder unsubstituierten Benzylrest darstellt und R² und R³ gleich oder verschieden sind und Wasserstoff, eine Alkylgruppe mit 1 bis 6 Kohlenstoffatomen oder eine Hydroxyalkylgruppe mit 2 bis 4 Kohlenstoffatomen bedeuten; und als Kupplersubstanz mindestens ein m-Phenylendiamin der allgemeinen Formel (II) 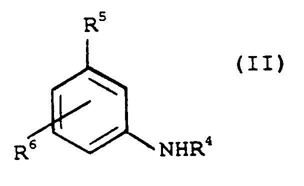 in der R⁴ gleich -CONH₂ oder -SO₂CH₃ ist, R⁵ gleich NR⁷R⁸ ist, wobei R⁷ und R⁸ gleich oder verschieden sein können und Wasserstoff oder eine Alkylgruppe mit 1 bis 4 Kohlenstoffatomen darstellen, und R⁶ Wasserstoff, eine Alkylgruppe mit 1 bis 4 Kohlenstoffatomen oder eine Alkoxygruppe mit 1 bis 4 Kohlenstoffatomen bedeutet; enthält.

2. Mittel nach Anspruch 1, dadurch gekennzeichnet, daß die Entwicklersubstanz der allgemeinen Formel (I) ausgewählt ist aus 4,5-Diamino-1-alkylpyrazol, 4,5 Diamino-1-benzylpyrazol, 4,5-Diamino-1-(4'-methylbenzyl)-pyrazol und 4,5-Diamino-1-(4'-chlorbenzyl)-pyrazol.

3. Mittel nach Anspruch 1 oder 2, dadurch gekennzeichnet, daß die Entwicklersubstanz der Formel (I) in einer Menge von 0,01 bis 4,0 Gewichtsprozent enthalten ist.

4. Mittel nach einem der Ansprüche 1 bis 3, dadurch gekennzeichnet, daß die Kupplersubstanz der allgemeinen Formel (II) ausgewählt ist aus N-(3-Dimethyl-amino)phenylharnstoff und 3-(N-Methylsulfonyl)amino-N,N-dimethylanilin.

5. Mittel nach einem der Ansprüche 1 bis 4, dadurch gekennzeichnet, daß die Kupplersubstanz der Formel (II) in einer Menge von 0,01 bis 4,0 enthalten ist.

6. Mittel nach einem der Ansprüche 1 bis 5, dadurch gekennzeichnet, daß es zusätzlich eine Farbkomponente enthält, die ausgewählt ist aus 6-Amino-2-methylphenol, 2-Amino-5-methylphenol, C. I. Basic Violet 14, C. I. Basic Violet 2, 2-Amino-4,6-dinitrophenol, 2-Amino-5-(2'-hydroxyethyl)amino-nitrobenzol, 2-Methylamino-5-bis-(2'-hydroxyethyl)aminonitrobenzol, C. I. Acid Brown 4, 1,4-Diaminoanthrachinon und 1,4,5,8-Tetraaminoanthrachinon.

7. Mittel nach einem der Ansprüche 1 bis 6, dadurch gekennzeichnet, daß es zusätzlich eine Entwicklersubstanz enthält, die ausgewählt ist aus 1,4-Diaminobenzol, 2,5-Diaminotoluol und 2,5-Diaminophenylethanol.

8. Mittel nach einem der Ansprüche 1 bis 7, dadurch gekennzeichnet, daß es zusätzlich eine Kupplersubstanz enthält, die ausgewählt ist aus Resorcin, 4-Chlorresorcin, 4,6-Dichlorresorcin, 2-Methylresorcin, 2-Amino-4-(2'-hydroxyethyl)amino-anisol, m-Phenylendiamin, 5-Amino-2-methylphenol, 2,4-Diaminophenoxy-ethanol, 4-Amino-2-hydroxyphenoxyethanol, 1-Naphthol, 3-Aminophenol, 3-Amino-2-methylphenol, 4-Hydroxy-1,2-methylendioxybenzol, 4-Amino-1,2-methylendioxybenzol, 2,4-Diamino-5-methylphenetol, 4-Hydroxy-indol und 3,5-Diamino-2,6-dimethoxypyridin.

9. Mittel nach einem der Ansprüche 1 bis 8, dadurch gekennzeichnet, daß es einen pH-Wert von 6,5 bis 11,5 aufweist.

10. Mittel nach einem der Ansprüche 1 bis 9, dadurch gekennzeichnet, daß es in Form einer wäßrigen oder wäßrig-alkoholischen Lösung, einer Creme, eines Gels oder einer Emulsion vorliegt.

## Claims

1. Agent for the oxidative dyeing of hair based on a developer substance-coupler substance combination, characterised in that it contains as developer substance at least one diaminopyrazole of the general formula (I) 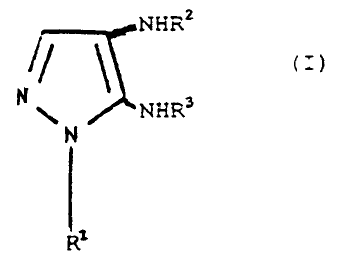 in which R¹ represents hydrogen, an alkyl group having from 1 to 6 carbon atoms, a hydroxyalkyl group having from 2 to 4 carbon atoms or a substituted or unsubstituted benzyl residue and R² and R³ are identical or different and represent hydrogen, an alkyl group having from 1 to 6 carbon atoms or a hydroxyalkyl group having from 2 to 4 carbon atoms; and as coupler substance at least one m-phenylenediamine of the general formula (II) 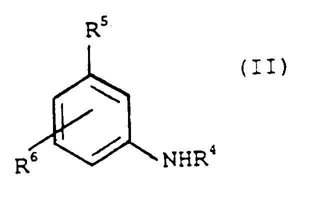 in which R⁴ is -CONH₂ or -SO₂CH₃, R⁵ is NR⁷R⁸, wherein R⁷ and R⁸ may be identical or different and represent hydrogen or an alkyl group having from 1 to 4 carbon atoms, and R⁶ represents hydrogen, an alkyl group having from 1 to 4 carbon atoms or an alkoxy group having from 1 to 4 carbon atoms.

2. Agent according to Claim 1, characterised in that the developer substance of the general formula (I) is selected from 4,5-diamino-1-alkylpyrazole, 4,5-diamino-1-benzylpyrazole, 4,5-diamino-1-(4'-methylbenzyl)-pyrazole and 4,5-diamino-1-(4'-chlorobenzyl)-pyrazole.

3. Agent according to Claim 1 or 2, characterised in that the content of developer substance of formula (I) is from 0.01 to 4.0 % by weight.

4. Agent according to any one of Claims 1 to 3, characterised in that the coupler substance of the general formula (II) is selected from N-(3-dimethylamino)phenylurea and 3-(N-methylsulphonyl)amino-N,N-dimethylaniline.

5. Agent according to any one of Claims 1 to 4, characterised in that the content of coupler substance of formula (II) is from 0.01 to 4.0.

6. Agent according to any one of Claims 1 to 5, characterised in that it additionally contains a dye component selected from 6-amino-2-methylphenol, 2-amino-5-methylphenol, C.I. Basic Violet 14, C.I. Basic Violet 2, 2-amino-4,6-dinitrophenol, 2-amino-5-(2'-hydroxyethyl)aminonitrobenzene, 2-methylamino-5-bis(2'-hydroxyethyl)aminonitrobenzene, C.I. Acid Brown 4, 1,4-diaminoanthraquinone and 1,4,5,8-tetraaminoanthraquinone.

7. Agent according to any one of Claims 1 to 6, characterised in that it additionally contains a developer substance selected from 1,4-diaminobenzene, 2,5-diaminotoluene and 2,5-diaminophenylethanol.

8. Agent according to any one of Claims 1 to 7, characterised in that it additionally contains a coupler substance selected from resorcinol, 4-chlororesorcinol, 4,6-dichlororesorcinol, 2-methylresorcinol, 2-amino-4-(2'-hydroxyethyl)amino-anisole, m-phenylenediamine, 5-amino-2-methylphenol, 2,4-diaminophenoxyethanol, 4-amino-2-hydroxyphenoxyethanol, 1-naphthol, 3-aminophenol, 3-amino-2-methylphenol, 4-hydroxy-1,2-methylenedioxybenzene, 4-amino-1,2-methylenedioxybenzene, 2,4-diamino-5-methylphenetole, 4-hydroxyindole and 3,5-diamino-2,6-dimethoxypyridine.

9. Agent according to any one of Claims 1 to 8, characterised in that it has a pH of from 6.5 to 11.5.

10. Agent according to any one of Claims 1 to 9, characterised in that it is in the form of an aqueous or aqueous-alcoholic solution, a cream, a gel or an emulsion.

## Revendications

1. Agent de teinture oxydative des cheveux, à base d'une combinaison substance révélatrice-substance de couplage, caractérisé en ce qu'il contient comme substance révélatrice au moins un diaminopyrazol de formule générale (I) 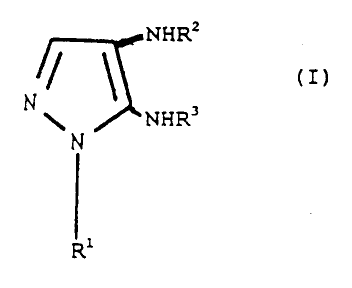 dans laquelle R¹ est l'hydrogène, un groupe alkyle avec de 1 à 6 atomes de carbone, un groupe hydroxyalkyle avec de 2 à 4 atomes de carbone ou bien un résidu benzyle substitué ou non substitué, et R² et R³ sont identiques ou différents et sont l'hydrogène, un groupe alkyle ayant de 1 à 6 atomes de carbone ou un groupe hydroxyalkyle ayant de 2 à 4 atomes de carbone, et contenant comme substance de couplage au moins un m-phénylènediamine de formule générale (II) 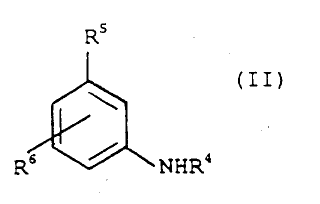 dans laquelle R⁴ est -CONH₂ ou -SO₂CH₃, R⁵ est NR⁷R⁸, R⁷ et R⁸ pouvant être identiques ou différents et sont l'hydrogène ou bien un groupe alkyle ayant de 1 à 4 atomes de carbone, et R⁶ étant de l'hydrogène, un groupe alkyle ayant de 1 à 4 atomes de carbone ou un groupe alkoxy ayant de 1 à 4 atomes de carbone.

2. Agent selon la revendication 1, caractérisé en ce que la substance révélatrice de la formule générale (I) est choisie parmi le 4,5-diamino-1-alkylpyrazol, 4,5-diamino-1-benzylpyrazol, 4,5-diamino-1-(4'-méthylbenzyl)-pyrazol et le 4,5-diamino-1-(4'-chlorobenzyl)-pyrazol.

3. Agent selon la revendication 1 ou 2, caractérisé en ce que la substance révélatrice de formule (I) est contenue en une quantité allant de 0,01 à 4,0 pour-cent en poids.

4. Agent selon l'une des revendications 1 à 3, caractérisé en ce que la substance de couplage de formule générale (II) est sélectionnée parmi la N-(3-diméthyl-amino)phénylurée et la 3-(N-méthylsulfonyl)amino-N,N-diméthylaniline.

5. Agent selon l'une des revendications 1 à 4, caractérisé en ce que la substance de couplage de formule (II) est contenue en une quantité de 0,01 à 4,0 pour-cent en poids.

6. Agent selon l'une des revendications 1 à 5, caractérisé en ce qu'il contient en plus un composant tinctorial choisi parmi
le 6-amino-2-méthyl phénol,
le 2-amino-5-méthylphénol,
C.l. Basic Violet 14,
C.l. Basic Violet 2,
le 2-amino-4,6-dinitrophénol,
le 2-amino-5-(2'-hydroxyéthyl)amino-nitrobenzène,
le 2-méthylamino-5-bis-(2'-hydroxyéthyl)-aminonitrobenzène,
C.l. Acid Brown 4,
la 1,4-diaminoanthraquinone, et
la 1,4,5,8-tétraamino-anthraquinone.

7. Agent selon l'une des revendications 1 à 6, caractérisé en ce qu'il contient en plus une substance révélatrice choisie parmi le 1,4-diaminobenzène, le 2,5-diaminotoluène et le 2,5-diaminophényléthanol.

8. Agent selon l'une des revendications 1 à 7, caractérisé en ce qu'il contient en plus une substance de couplage choisie parmi
la résorcine,
la 4-chlorrésorcine,
la 4,6-dichlorrésorcine,
la 2-méthylrésorcine,
le 2-amino-4-(2'-hydroxyéthyl)amino-anisol,
la m-phénylènediamine,
le 5-amino-2-méthylphénol,
le 2,4-diaminophénoxy-éthanol,
le 4-amino-2-hydroxyphénoxyéthanol,
le 1-naphtol,
le 3-aminophénol,
le 3-amino-2-méthylphénol,
le 4-hydroxy-1,2-méthylènedioxybenzène,
le 4-amino-1,2-méthylènedioxybenzène,
le 2,4-diamino-5-méthyphénétol,
le 4-hydroxy-indol, et
la 3,5-diamino-2,6-diméthoxypyridine.

9. Agent selon l'une des revendications 1 à 8, caractérisé en ce qu'il présente une valeur de pH comprise entre 6,5 et 11,5.

10. Agent selon l'une des revendications 1 à 9, caractérisé en ce qu'il se présente sous la forme d'une solution aqueuse ou alcoolo-aqueuse, d'un crème, d'un gel ou d'une émulsion.
